Europäisches Patentamt

(19) **European Patent Office**

**Office européen des brevets**

(11) Numéro de publication: **0 190 061**
**A1**

(12) ## DEMANDE DE BREVET EUROPEEN

(21) Numéro de dépôt: **86400033.6**

(22) Date de dépôt: **09.01.86**

(51) Int. Cl.⁴: **C 12 Q 1/00**
**A 01 C 1/00**

(30) Priorité: **10.01.85 FR 8500303**

(43) Date de publication de la demande:
**06.08.86 Bulletin 86/32**

(84) Etats contractants désignés:
**AT BE CH DE FR GB IT LI LU NL SE**

(71) Demandeur: **INSTITUT NATIONAL DE LA RECHERCHE AGRONOMIQUE (INRA)**
**149, rue de Grenelle**
**F-75341 Paris Cedex 07(FR)**

(72) Inventeur: **Tepfer, David**
**6, Passage de la Main d'Or**
**F-75011 Paris(FR)**

(72) Inventeur: **Yacoub, Boutro Adly**
**8, rue de Londres**
**F-67230 Benfeld(FR)**

(74) Mandataire: **Warcoin, Jacques**
**Cabinet Régimbeau 26, avenue Kléber**
**F-75116 Paris(FR)**

(54) **Modèle de rhizosphère utile notamment pour l'étude des parasites des plantes.**

(57) L'invention concerne un modèle de rhizosphère utile notamment pour l'étude de composés ayant une activité au niveau de la rhizosphère et/ou des racines d'une plante, caractérisé en ce qu'il est composé d'au moins deux compartiments:

– un compartiment *a* contenant un milieu nutritif assurant le développement de racines de ladite plante transformées par tout ou partie de Ri T-ADN,

– un compartiment *b* comportant un milieu support des racines transformées du compartiment *a*,

les deux compartiments étant disposés de façon que les racines transformées croissant dans le compartiment *a* puissent s'étendre dans le compartiment *b*.

EP 0 190 061 A1

0190061

MODELE DE RHIZOSPHERE UTILE NOTAMMENT POUR L'ETUDE DES PARASITES
DES PLANTES.

La présente invention concerne un modèle de
rhizosphère utile, notamment, pour l'étude de produits
actifs sur les racines des plantes et/ou leurs parasites.
La rhizosphère au sens strict désigne
l'ensemble des régions du sol qui sont en contact
avec les racines des plantes supérieures, la surface
même des racines est dénommée rhizoplant.
Dans la présente description, on entendra
déginer par "rhizosphère" aussi bien la rhizosphère
proprement dite que le rhizoplant.
La rhizosphère est un milieu riche en
microorganismes dont les interactions avec les
plantes sont difficiles à étudier expérimentalement.
Ces difficultés tiennent essentiellement :
. au grand nombre d'organismes différents qui sont
présents ;
. au fait que les relations dans l'espace sont
détruites lorsque le sol est bouleversé ; et

. à la fragilité de certaines structures concernées (radicelles ou hyphes fongiques ; et

. à l'influence complexe des parties aériennes des plantes sur la physiologie des racines et des rhizomes.

La présente invention concerne un modèle de rhizosphère réalisable _in vitro_ et permettant de travailler dans des conditions parfaitement déterminées et reproductibles.

Plus particulièrement, il s'agit d'un modèle de rhizosphère utile notamment pour l'étude de composés ayant une activité au niveau de la rhizosphère et/ou des racines d'une plante, caractérisé en ce qu'il est composé d'au moins deux compartiments :

. un compartiment a contenant un milieu nutritif assurant le développement de racines de ladite plante transformées par tout ou partie de RiT-ADN ;

. un compartiment b comportant un milieu support des racines transformées du compartiment a ;

les deux compartiments étant disposés de façon que les racines transformées croîssant dans le compartiment a puissent s'étendre dans le compartiment b.

La transformation des racines par tout ou partie de RiT-ADN est effectuée, de préférence, en utilisant des souches de la bactérie Agrobacterium rhizogenes.

A. rhizogenes qui est une bactérie du sol provoque la formation de racines sur les plantes supérieures grâce à un épisome bactérien, le plasmide Ri ("Root inducing").

Les racines induites par A. rhizogenes présentent plusieurs particularités. Ainsi les racines s'allongent à une vitesse beaucoup plus importante que les racines normales et elles produisent des racines latérales avec une fréquence élevée. Ces particularités conduisent à la formation d'un chevelu abondant de racines adventives ("Hairy Roots").

Bien qu'il soit, en général, plus simple d'utiliser pour la transformation la bactérie A. rhizogenes, il est également possible d'utiliser une autre bactérie dans laquelle on a transféré le T-DNA du plasmide Ri ou sa séquence active par des techniques biologiques connues.

On peut induire la transformation à partir de nombreux matériaux provenant des plantes, par exemple à partir de graines, de fragments de tiges ou de fragments de racines. En général, la transformation effectuée, on préférera reprendre une culture pure et aseptique à partir d'un fragment de racine transformée pour placer le modèle dans les meilleures conditions de reproductibilité.

Les milieux de culture mis en oeuvre peuvent être très variés et ont déjà été décrits.

Dans le modèle proposé, les parties aériennes de la plante sont modélisées par un milieu riche qui nourrit les racines à leur extrémité proximale, tandis que les extrémités distales placées dans le second compartiment sont en contact avec un milieu nutritif pauvre qui simule les conditions pouvant se rencontrer dans le sol.

A titre d'exemple, on peut dire que les milieux synthétiques (variables selon l'espèce) mis en oeuvre peuvent être les suivants :

milieu riche : Murashige T., Skoog F., Physiol. Plant 15, 473-497 (1962),

milieu pauvre: - agar    6 g/l
                 - $H_2O$ bidistillée
                 - tamponné, pH 7,0
                 (par exemple : tourbe stérile traitée avec du $CaCO_3$ à 1 % pour ramener le pH à 7,0).

Ce modèle peut être aisément réalisé sur le plan pratique avec deux boîtes de Pétri ou récipients équivalents ayant des dimensions suffisamment différentes pour que l'un des récipients puisse être placé dans l'autre. Dans ces conditions, le récipient intérieur peut devenir le compartiment a et le récipient extérieur le compartiment b.

Ce type de modèle très simple permet de prévoir un grand nombre de modèles sur une surface restreinte, ce qui permet une sélection ou une étude systématique très complète sans avoir recours à des surfaces de serres ou de champs importantes dans lesquelles, d'ailleurs, les conditions ne sont pas forcément reproductibles ou parfaitement contrôlables.

Le modèle proposé présente, en outre, de nombreux autres avantages. En particulier, il est possible de soumettre les racines à des contraintes diverses en faisant varier la composition du milieu de croissance, alors que cela est beaucoup plus difficile lorsqu'il faut agir sur la plante elle-même dans le cas de cultures en serre.

Enfin, ce modèle se prête bien aux techniques autoradiographiques et permet d'étudier les interactions hôte/microorganisme  et la circulation de certains composés marqués.

Il s'agit là d'une propriété particulièrement intéressante dans l'étude de certains composés à activité systémique.

Bien que le modèle de rhizosphère selon la présente invention puisse être utilisé comme instrument d'étude et de recherche sur les interactions entre la rhizosphère et les racines par exemple, le modèle selon l'invention est plus particulièrement utilisable dans l'étude des parasites des plantes :

. pour obtenir des inoculums standards dans le cas de microorganismes qui ne peuvent être cultivés in vitro par d'autres méthodes, et

. pour effectuer une sélection des produits susceptibles de lutter contre certains microorganismes, dans des conditions acceptables financièrement, en particulier lorsqu'il s'agit de produits à action systémique, qui ne peuvent que très difficilement être testés sur des milieux de culture normaux.

Suivant l'étude que l'on désire poursuivre, il est possible d'effectuer une infection, soit dans le compartiment a pour les parasites qui se propagent à partir des parties aériennes de la plante, soit dans le compartiment b pour étudier les parasites de la rhizosphère. Il est même possible d'étudier les transmissions de parasites entre les racines de deux plantes en prévoyant deux compartiments a dans le compartiment b.

Suivant la nature du parasite, celui-ci peut, dans certains cas, être déjà présent dans les fragments de racines qui seront transformés.

Actuellement, l'étude systématique de la rhizomanie des betteraves et la recherche d'un fongicide permettant de lutter contre cette maladie sont limitées par la quasi impossibilité de cultiver in vitro ce champignon.

La rhizomanie des betteraves est une maladie caractérisée par une prolifération anarchique des radicelles et une apparition de nécrose qui détruit les betteraves dans l'est de la France, en Italie, au Japon, etc.

Les études poursuivies ont montré que cette maladie est d'origine virale, cette virose étant transmise par un champignon du genre _Polymyxa_ dénommé _Polymyxa betae_.

Alors que jusqu'à maintenant il n'avait pas été possible d'obtenir des cultures _in vitro_ de ce champignon, le modèle de rhizosphère selon la présente invention a permis, enfin, une culture et une étude plus fine de la physiologie de ce champignon _Polymyxa betae_.

D'ailleurs, en tout état de cause, même si une culture _in vitro_ pouvait être réalisée, il ne serait pas possible de tester de façon satisfaisante des produits à action systémique qui sont pourtant particulièrement intéressants lorsque l'agent pathogène s'attaque aux racines d'une plante.

Bien que les exemples ci-après soient plus particulièrement décrits en se référant à _Polymyxa betae_, le modèle de rhizosphère selon l'invention est utilisable pour l'étude de nombreux pathogènes des racines qui ne peuvent être cultivés _in vitro_.

Ainsi, on peut envisager d'utiliser le modèle selon l'invention pour l'étude des agents du mildiou de la vigne et du tournesol ou bien de la hernie des Crucifères, dans la mesure où de nombreuses plantes dont les racines induites par _A. rhizogenes_ peuvent être cultivées _in vitro_ sont également réceptives à ces parasites.

Il est également possible d'utiliser d'autres plantes telles que <u>Convolvulus sepium</u> et <u>Convolvulus arvensis</u> ou bien <u>Ipomoea batatas</u> qui sont réceptives à <u>A. rhizogenes</u> et qui sont attaquées par de nombreux parasites, en particulier <u>Fusarium, Ceratosystis</u> et <u>Rotylenchulus</u>.

Les exemples ci-après, décrits en se référant aux figures, permettront de mettre en évidence d'autres caractéristiques et avantages de la présente invention, sans, bien entendu, la limiter.

EXEMPLE 1

<u>Préparation de racines transformées</u>

On utilise pour transformer des racines de betteraves deux souches d'<u>Agrobacterium rhizogenes</u> : <u>A. rhizogenes</u> souche A4 et <u>A. rhizogenes</u> 8196.

La souche 8196 est déposée à la collection de souches NCPPB (National Collection of Plants Pathogenic Bacteria - Plants Pathology Laboratories - Grande-Bretagne).

Ces souches sont cultivées sur un milieu conventionnel (Petit A. et al., Mol. Gen. Genet. 190, 204-214 (1983).

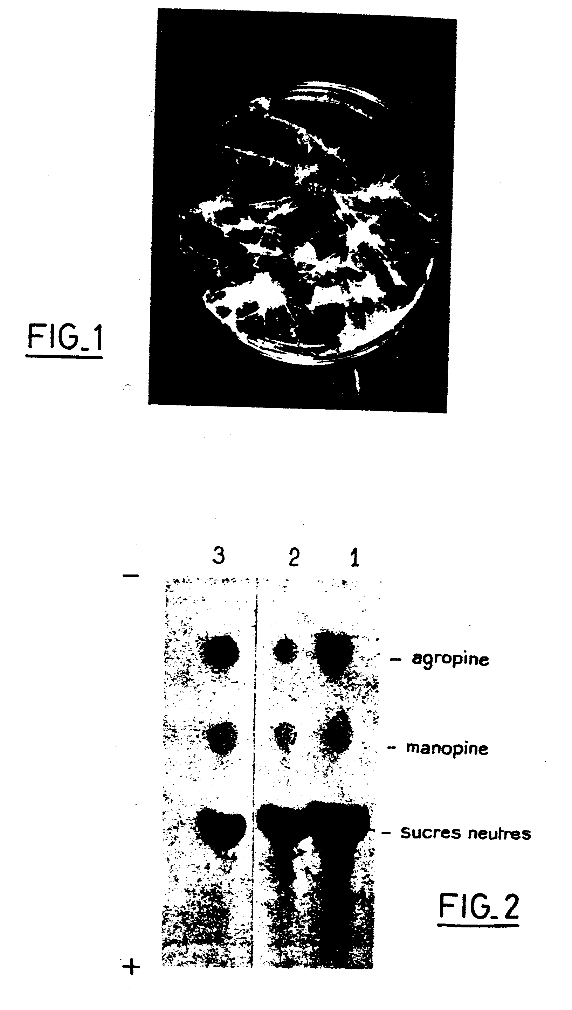

La transformation est effectuée selon les processus connus en la matière, à savoir : les fragments de betteraves préalablement stérilisés sont inoculés avec $10^9$ bactéries dans 0,1 ml d'eau. Après 10-21 jours des racines apparaissent. Des cultures de ces racines de betteraves transformées par Ri T-ADN de <u>A. rhizogenes</u> souche A4 présentent les phénotypes transformés (fig. 1) qui ont déjà été rapportés pour diverses autres espèces (Tepfer, 1984: vitesse de croissance rapide, taux élevé de branchement et résistance générale aux contraintes extérieures.

Dans le cas de racines de la betterave, aucune pousse n'est régénérée à partir de cette culture de racines, que ce soit in vitro ou lorsque les racines sont cultivées dans des pots.

Pour les souches transformées avec A. rhizogenes souche A4, la production de mannopine et agropine démontre qu'elles contiennent et expriment Ri T-ADN (fig. 2).

Sur la figure 2, les lignes sont les suivantes :

ligne 1 : racines de betterave provoquées par la souche A4 d'A. rhizogenes (10 µl extrait),

ligne 2 : idem ligne 1 mais 5 µl extrait,

ligne 3 : témoin, tabac transformé par A. tumefaciens A60 productrice d'agropine et manopine (5 µl d'extrait).

EXEMPLE 2

Infection des cellules transformées

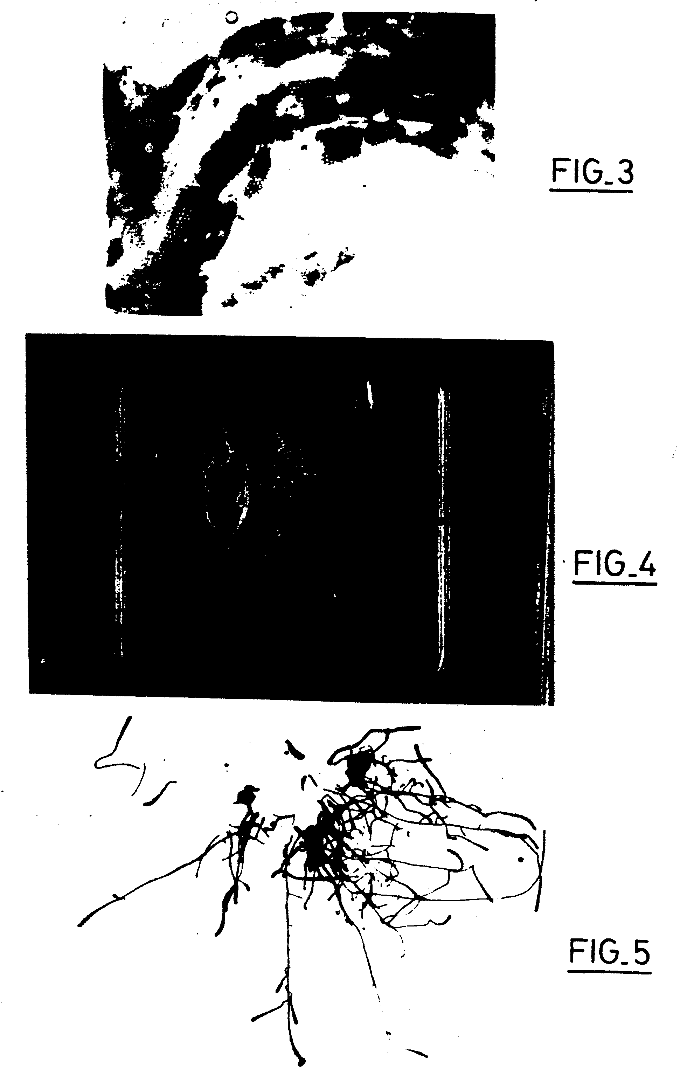

On prépare une solution destinée à l'infection par P. betae à partir de racines de betteraves contaminées dans lesquelles on observe les cystosori (fig. 3) qui sont l'un des spores typiques de réserve du champignon.

Après contamination, à la fois la plante donneuse et les racines transformées infectées in vitro contiendront ces structures. Les zoospores flagellés seront libérés à partir des racines transformées par macération.

Ceci sera considéré comme une démonstration de l'infection des racines de betteraves transformées par P. betae.

Le modèle de rhizosphère selon la présente invention utilisé est représenté à la figure 4.

Le compartiment intérieur 1 contient un milieu ayant la composition suivante :

- sels de Murashige et Skoog
- vitamines de Morel (Morel G. et Wetmore R. (1961), Am. J. Bot. 38, 141-143
- Fe EDTA et sucrose.

Il s'agit là d'un milieu riche.

Quant au compartiment extérieur 2, il comporte un milieu de composition :

- agar  6 g/l (on peut ajouter 1,66 g/l de gelite)
- 1 cuillérée/1 de tourbe stérile (ramenée à
  pH 7,0 avec CaCO$_3$).
Il s'agit du milieu pauvre.

Les racines transformées préparées à l'exemple 1
sont mises en croissance dans ce système à deux
compartiments, l'extrémité distale étant tout d'abord
disposée dans le compartiment a et au cours du
développement les racines transformées coloniseront
le milieu et le compartiment b.

Des fragments de racines de plantes contenant
Cystosori sont stérilisés en surface et mis à macérer
dans de l'eau distillée stérile. Des gouttes de 20 µl
contenant des fragments de racine sont déposées sur
les racines transformées dans le compartiment externe.

Diverses infectionsdues à des microorganismes
contaminant et à des fungis endophytiques autres
que P. betae se développent en plusieurs jours.

Ces infections sans intérêt se développement
lentement à cause du manque de source carbonée dans
le compartiment externe. Ceci permet par inspection
sous microscope de définir des zones du disque de
culture qui sont libres d'infection secondaire et où
les fragments de racines sont clairement infectés
par P. betae. Ces zones de culture sont transférées
sur des nouveaux disques de culture à proximité de
racines transformées non infectées et on observe
l'infection de ces racines. Ces cultures sont incubées
et une macération de ces racines infectées nouvelles
est utilisée pour infecter une nouvelle série de
cultures à deux compartiments, de même que des cultures
de racines de betteraves qui poussent dans un seul
compartiment sur un milieu riche. Les racines infectées
avec P. betae apparaissent saines et présentent une
excellente croissance.

On a ainsi pu propager l'infection de p. betae depuis 6 mois à l'aide d'environ 20 transferts. Une fois établie en double boîtes, la culture du champignon peut se poursuivre sur racines qui poussent sur milieu riche uniquement, les racines peuvent pousser dans un milieu liquide et augmenter la quantité d'inoculum dans un fermenteur.

EXEMPLE 3

Utilisation de l'autoradiographie pour étudier les interactions champignon/racine in vitro

Des composés marqués ont été utilisés très largement dans l'étude des systèmes biologiques au niveau subcellulaire, cellulaire et au niveau de l'organisme.

Le modèle de rhizosphère selon la présente invention se prête particulièrement bien à des études de type autoradiographique.

Les composés marqués peuvent être placés soit dans le compartiment externe, soit dans le compartiment interne et la distribution à l'intérieur des racines et des microorganismes associés est très facile à observer par autoradiographie. En effet, à la fin de la période de marquage, le compartiment interne est enlevé après avoir découpé les racines qui assurent le contact entre les deux compartiments.La boîte de Pétri ouverte est placée à l'envers sur un filtre de papier et son contenu, c'est-à-dire le milieu externe, est posé sur le papier filtre en injectant de l'air dans des petits trous qui sont faits derrière la boîte de Pétri. Le milieu externe est couvert avec un film de PVC et séché jusqu'à obtenir une couche très fine en utilisant des techniques conventionnelles. L'autoradiographie est effectuée en plaçant un film sensible aux rayonnements X contre le film de PVC.

A titre d'exemple, la figure 5 montre que les racines de Calystegia sepium prélèvent l'isotope dans le compartiment central et que dans le cas de $S^{35}$ celui-ci n'est pas excrété dans le milieu support.

Cette technique peut servir pour étudier les relations de métabolisme entre l'hôte et le micro-organisme, mais peut également servir pour suivre la translocation de composés marqués tels que des fongicides.

EXEMPLE 4

Des essais équivalents ont été réalisés avec des racines transformées des espèces suivantes :

- le tabac
- la carotte
- Calystegia arvensis
- Calystegia sepium
- Nicotiana plumbaginifolia
- la pomme de terre
- la betterave à sucre
- le colza
- le topinambour
- le haricot vert
- l'endive
- l'alfalfa
- la tomate
- le tournesol.
- la betterave potagère
- chou chinois
- petit pois
- fenouil
- concombre

0190061

## REVENDICATIONS

1) Modèle de rhizosphère utile notamment pour l'étude de composés ayant une activité au niveau de la rhizosphère et/ou des racines d'une plante, caractérisé en ce qu'il est composé d'au moins deux compartiments :

- un compartiment a contenant un milieu nutritif assurant le développement de racines de ladite plante transformées par tout ou partie de Ri T-ADN ;

- un compartiment b comportant un milieu support des racines transformées du compartiment a ;

les deux compartiments étant disposés de façon que les racines transformées croissant dans le compartiment a puissent s'étendre dans le compartiment b.

2) Modèle de rhizosphère selon la revendication 1, caractérisé en ce que le compartiment a contient un milieu nutritif plus riche que le milieu du compartiment b.

3) Modèle de rhizosphère selon l'une des revendications 1 et 2, caractérisé en ce que les racines utilisées contiennent de l'ADN provenant du T-DNA du plasmide d'Agrobacterium rhizogenes.

4) Modèle de rhizosphère selon l'une des revendications 1 à 3, caractérisé en ce que les racines transformées du compartiment a sont des racines de betterave.

5) Modèle de rhizosphère selon la revendication 4, caractérisé en ce que les racines sont infectées par Polymyxa betae.

FIG.1

– agropine

– manopine

– Sucres neutres

FIG.2

0190061

20/1290061

FIG. 3

FIG. 4

FIG. 5

**0190061**

Numéro de la demande

Office européen
des brevets

**RAPPORT DE RECHERCHE EUROPEENNE**

EP 86 40 0033

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| A | NATURE, vol. 295, no. 5848, 4 février 1982, pages 432-434, Macmillan Journals Ltd., Chesham, Bucks, GB; M.-D. CHILTON et al.: "Agrobacterium rhizogenes inserts T-DNA into the genomes of the host plant root cells" * En entier * | 3 | C 12 Q 1/00 A 01 C 1/00 |
| A | WO-A-8 403 298 (RESEARCH AND DEVELOPMENT INSTITUTE) | | |
| A | EP-A-0 122 791 (AGRIGENETICS RESEARCH ASSOCIOATES LTd.) | | |

---

| | DOMAINES TECHNIQUES RECHERCHES (Int. Cl 4) |
|---|---|
| | C 12 Q A 01 C C 12 N C 12 M |

Le présent rapport de recherche a été établi pour toutes les revendications

| Lieu de la recherche | Date d'achèvement de la recherche | Examinateur |
|---|---|---|
| LA HAYE | 17-04-1986 | MEYLAERTS H. |